# FASCICULE DE BREVET EUROPEEN

(11) **EP 0 345 109 B1**
(45) Date de publication et mention de la délivrance du brevet: **15.09.1993**
(21) Numéro de dépôt: 89401306.9
(22) Date de dépôt: 11.05.1989
(51) Int. Cl.: C01B 33/193, A61K 9/18

(54) **Silice précipitée absorbante et composition à base de cette silice**
Absorbierende Fällungskieselsäure und Zusammensetzung auf der Basis dieser Kieselsäure
Absorbing precipitated silica and composition based thereon

(30) Priorité: 19.05.1988 FR 8806731
(43) Date de publication de la demande: 06.12.1989
(73) Titulaire: RHONE-POULENC CHIMIE, 92408 Courbevoie Cédex (FR)
(72) Inventeur: Ponchon, Jean-Luc, F-69290 - Craponne (FR); Rabute, Lionel, F-69009 - Lyon (FR)
(74) Mandataire: Dubruc, Philippe

(56) Documents cités:
- US-A- 2 924 510
- US-A- 3 445 189
- US-A- 3 967 563

## Description

La présente invention concerne une silice précipitée absorbante et les compositions à base de cette silice.

Il est bien connu de conditionner des liquides sur un support silice. Ce conditionnement a pour but de transformer un liquide non ou difficilement manipulable en poudre fluide pouvant être stockée facilement, par exemple en sac, et pouvant aussi se disperser aisément et bien se mélanger à d'autres constituants solides divisées.

Les compositions ainsi obtenues : liquide absorbé sur un support silice, seront dans la suite du texte appelées compositions conditionnées.

Ces compositions doivent présenter une teneur élevée en matière active ainsi qu'une forte densité. Elles doivent pouvoir être manipulées facilement ce qui implique une bonne coulabilité et un faible poussiérage. Ces différentes exigences sont parfois contradictoires. Ces qualités dépendent bien entendu étroitement de la nature de la silice utilisée comme support.

US-A-3 445 189 mentionne l'utilisation d'une silice comme support pour les liquides, ladite silice présentant une surface spécifique de 100 à 250 m²/g et une prise d'huile de plus de 200 ml/100 g.

Le premier objet de l'invention est la mise au point d'une silice absorbante de qualité améliorée.

Le second objet de l'invention est la préparation d'une composition conditionnée à densité élevée, à coulabilité améliorée et à poussiérage réduit.

Dans ce but la silice précipitée de l'invention présente les caractéristiques suivantes :
- surface BET d'au moins 170 m²/g;
- prise d'huile (DOP) comprise entre 220 et 300 ml/100g ;
- densité de remplissage à l'état tassé d'au moins 0,29 ;
- diamètre médian des particules compris entre 80 et 150 µm ;
- un indice de dispersion granulométrique d'au plus 0,70.

On a pu constater que grâce à l'ensemble des caractéristiques mentionnées ci-dessus, la silice de l'invention constituait un support particulièrement bien adapté pour les liquides.

Bien entendu, l'invention concerne aussi une composition conditionnée à base d'un liquide adsorbé sur le support comprenant de la silice précipitée telle que définie ci-dessus.

D'autres caractéristiques et avantages de l'invention seront mieux compris à la lecture de la description et des exemples concrets mais non limitatifs qui vont suivre.

La silice qui a été définie plus haut présente ses propriétés particulièrement intéressantes grâce à la combinaison de caractéristiques qui vont être étudiées plus précisément ci-dessous.

Tout d'abord, cette silice présente une surface BET d'au moins 170 m2/g, plus particulièrement d'au moins 210 m²/g. Notamment cette surface BET peut être plus particulièrement comprise entre 170 et 400 m2/g ou 210 et 400 m³/g.

La surface BET est déterminée selon la méthode de BRUNAUER-EMMET-TELLER décrite dans the Journal of the American Chemical Society vol. 60, page 309, February 1938 et selon la norme NF T45007 (5.11).

Une autre caractéristique de la silice de l'invention est sa prise d'huile. Cette prise d'huile (DOP), déterminée selon la norme NFT 30-022 (mars 53) en mettant en oeuvre le phtalate de dioctyle, est comprise entre 220 et 300 ml/100 g, notamment entre 220 et 280 et plus particulièrement entre 240 et 280 ml/100 g.

Sa densité est d'au moins 0,29. Il s'agit là de la densité de remplissage à l'état tassé (DRT) selon la norme AFNOR N° 030100. Plus particulièrement, la densité pourra être comprise entre 0,29 et 0,4 et notamment 0,29 et 0,36.

Enfin, la granulométrie est aussi une caractéristique importante de la silice de l'invention.

Cette granulométrie est telle que le diamètre médian des particules d₅₀ soit compris entre 80 et 150 µm, plus particulièrement entre 90 et 130 µm.

Cette granulométrie est déterminée par refus au tamis à sec selon la norme NF X11.507.

Par ailleurs la granulométrie de la silice de l'invention est réserrée. Ceci peut se traduire par un indice de dispersion (I.D) d'au plus 0,70, plus particulièrement d'au plus 0,6. Cet indice correspond au rapport (d84 -d16)/2d50 dans lequel dn est le diamètre pour lequel on a n% de particules de taille inférieure à cette valeur de diamètre.

Selon des modes de réalisation plus particuliers de l'invention, la silice peut présenter des caractéristiques supplémentaires.

La silice selon l'invention a généralement un pH compris entre 4 et 8 et plus particulièrement entre 5,5 et 7. Ce pH est celui déterminé selon la norme NFT-45007 (5.5).

Par ailleurs, la silice de l'invention a de préférence une perte au feu d'au plus 13 % sur produit tel quel et d'au plus 6 % sur produit sec [norme NFT 45007 (5.3)]
La silice peut être précipitée par toute méthode connue telle que par réaction d'un silicate avec un agent acidifiant, par exemple un acide inorganique du type acide carbonique, sulfurique ou chlorhydrique. C'est ainsi que l'on pourra utiliser des procédés avec addition d'acide sur un pied de cuve de silicate ou avec addition simultanée totale ou partielle d'acide et de silicate sur un pied de cuve d'eau en présence de sel ou non ou de solution de silicate.

On peut aussi avantageusement procéder à un post-traitement c'est-à-dire introduire dans le milieu réactionnel après une première précipitation une solution de silicate et/ou un acide.

Après filtration de la bouillie réactionnelle on récupère un gâteau de filtration que l'on lave éventuellement. Le gâteau est ensuite généralement délité pour être séché.

Lors du délitage et éventuellement à toute étape antérieure du procédé de préparation de la silice, il est intéressant d'ajouter au gâteau ou au milieu réactionnel un composé d'aluminium qui rend la suspension moins visqueuse et plus facilement pompable. On pourra se référer à ce sujet au brevet français N° 2.536.380.

On doit se mettre dans des conditions telles que le gâteau notamment à l'état délité présente avant séchage une perte au feu d'au plus 77 % de préférence d'au plus 75 %.

Il est à noter que l'on peut obtenir cette valeur de perte au feu par addition au gâteau, notamment lors de son délitage, de silice préalablement séchée.

Le séchage du gâteau pour obtenir le produit final peut se faire par tout moyen connu tel que, par exemple, par atomisation et plus particulièrement avec un atomiseur à turbine.

La silice de l'invention convient particulièrement bien au conditionnnement des liquides et donc à la préparation de compositions conditionnées.

Un très grand nombre de liquides peuvent être mis ainsi sur support. On peut citer à titre d'exemples les acides organiques ; les tensio-actifs utilisés en détergence du type anionique tels que les sulfonates ou du type non ionique tels que les alcools ou les phénols ; les accélérateurs de vulcanisation et les antioxydants pour l'industrie du caoutchouc.

On peut mentionner aussi plus particulièrement les liquides utilisables comme complément d'alimentation humaine et animale.

On citera notamment les vitamines et dans cette catégorie les vitamines A, B, C, D, E et K.

Dans le groupe des vitamines B on peut mentionner plus particulièrement la choline et ses dérivés notamment le chlorhydrate de choline.

L'absorption du liquide par le support silice se fait d'une manière connue en soi, par exemple par pulvérisation du liquide sur la silice dans un mélangeur.

La quantité de liquide absorbé sera fonction de l'application recherchée. La silice de l'invention permet d'obtenir des compositions à une teneur en liquide pouvant dépasser 50 % en poids, notamment jusqu'à 70 % et par exemple comprise entre 50 et 60.

D'une manière générale, la silice selon l'invention confère aux compositions conditionnées une densité élevée, une très bonne coulabilité et un poussiérage réduit.

A titre d'exemple, dans le cas particulier des compositions à base de vitamines, notamment de vitamine E et chlorhydrate de choline, la DRT des compositions peut être d'au moins 0,60, plus particulièrement d'au moins 0,65 et même d'au moins 0,70.

L'angle de talus des compositions (selon la norme NFT 20-221) est d'au plus 32°.

L'indice de poussiérage de ces mêmes compositions est inférieur à 2, voire même inférieur à 1.

Cette mesure du poussiérage se fait par l'appareil décrite dans la demande de brevet français n° 8703159 dont l'ensemble de l'enseignement est incorporé ici.

L'appareil est couplé à un système à micro-courant constant transformant la variation de résistance de la cellule photoélectrique en tension, transmise graphiquement sous forme d'un pic sur une table traçante graduée de 1 à 100 mV. La valeur en mV du pic constitue l'indice de poussiérage (IP).

A titre de comparaison, des compositions du même type mais sur des supports en silice de l'art antérieur présentent un IP d'au moins 3.

Les composition conditionnées avec vitamines présentent de préférence un indice de dispersion tels que défini plus haut d'au plus 0,55.

Des exemples concrets vont maintenant être donnés.

### EXEMPLE 1

Cet exemple concerne la préparation d'une silice absorbante selon l'invention.

On ajoute de l'acide sulfurique à un pied de cuve de silicate de sodium de rapport 3,5 à une température de 70°C. Le pH de la bouillie de silice formée est amené à la valeur 5. La bouillie est ensuite filtrée et le gâteau humide obtenu est lavé puis délité.

Lors du délitage on ajoute de la silice préalablement séchée de manière à obtenir un gâteau délité présentant une perte au feu de 75 %. Le pH est ensuite ajusté à 6,0.

Le gâteau délité est séché par atomisation. On obtient une silice présentant les caractéristiques ci-dessous :

| | | | |
|---|---|---|---|
| pH | 6,7 | DOP | 250 ml/100g |
| DRT | 0,32 | BET | 270 m²/g |
| D₅₀ | 105 µm | ID | 0,5 |

### EXEMPLE 2

On utilise comme support pour la vitamine E la silice de l'exemple 1.

La mise sur support de la vitamine se fait dans un mélangeur en V de 7 litres tournant à 20 T/mn avec un axe intérieur tournant à 1900 T/mn, muni de plaques au travers desquelles est pulvérisée la vitamine et sur lesquelles sont fixées des couteaux émotteurs.

On charge la totalité de la silice décrite ci-dessus dans le mélangeur puis on pulvérise la vitamine sur la silice. On mélange pendant 15 minutes puis on homogénéise pendant deux autres minutes.

La vitamine est incorporée à une température de 70°C et à un débit constant de 100 ml/mn.

Dans le mélange final, les proportions sont en poids de 46 % en SiO₂ et 54 % en acétate de d,l-alpha tocophérol.

On obtient une composition présentant les caractéristiques suivantes :

| | | |
|---|---|---|
| DRT | 0,70 | Angle de talus 30° |
| d₅₀ | 90 µm | IP ≦ 1 |
| ID | 0,55 | |

### EXEMPLE 3

Cet exemple décrit la préparation d'une seconde silice absorbante selon l'invention.

On procède comme dans l'exemple 1. Toutefois, à la fin de la réaction on procède à un traitement complémentaire en introduisant dans le milieu réactionnel une solution de silicate de sodium et d'acide sulfurique.

On lave et délite le gâteau et on ajoute de la silice préalablement séchée, de manière à obtenir une perte au feu du gâteau délité de 74 %. On ajoute aussi au gâteau de l'aluminate de sodium (4000 ppm en poids par rapport à la silice anhydre) ce qui rend la suspension de délitage plus pompable. Le pH est ajusté à 6 et la suspension séchée comme dans l'exemple 1.

La silice obtenue a les caractéristiques ci-dessous :

| | | | | | |
|---|---|---|---|---|---|
| pH | 7,0 | ID | 0,5 | d₅₀ | 100 µm |
| DRT | 0,34 | DOP | 240 ml/100g | BET | 220 m²/g |

### EXEMPLE 4

On procède à une mise sur support comme dans l'exemple 2 mais avec du chlorhydrate de choline sur la silice de l'exemple 3.

Les proportions dans la composition finale sont les suivantes : 60 parties en poids d'une solution aqueuse de chlorhydrate de choline à 70 %, 40 parties de silice, 0,3 partie d'additif (stéarate de magnésium).

La composition présente les caractéristiques ci-dessous :

| | | | |
|---|---|---|---|
| DRT | 0,77 | IP | 1,6 |
| Angle de talus | 31° | ID | 0,5 |
| d₅₀ | 90 µm | | |

### EXEMPLE 5 comparatif

On utilise comme support une silice vendue part la Société RHONE-POULENC sous la marque Tix-O-Sil 38A et présentant les caractéristiques ci-dessous :

| | | | |
|---|---|---|---|
| pH | 7 | ID | > 0,7 |
| DRT | 0,22 | DOP | 340 ml/100g |
| d₅₀ | 60 µm | BET | 330 m²/g |

On prépare une composition à base de vitamine E dans les mêmes conditions qu'à l'exemple 2, qui possède les caractéristiques suivantes :

| | | | |
|---|---|---|---|
| DRT | 0,50 | Angle de talus | 41° |
| d₅₀ | 80 µm | IP | > 5 |
| ID | > 0,7 | | |

On constate que par rapport à l'exemple 1, cette composition est d'un coulabilité plus faible et qu'elle est beaucoup plus poussiérante.

## Revendications (Revendications pour l'(les) Etat(s) contractant(s) suivant(s): AT, BE, CH, DE, FR, GB, GR, IT, LI, LU, NL, SE)

1. Silice précipitée présentant les caractéristiques suivantes :
- surface BET d'au moins 170 m²/g ;
- prise d'huile (DOP) comprise entre 220 et 300 ml/100g ;
- densité de remplissage à l'état tassé d'au moins 0,29 ;
- diamètre médian des particules compris entre 80 et 150 µm ;
- un indice de dispersion granulométrique d'au plus 0,70.

2. Silice selon la revendication 1 caractérisée en ce que sa surface BET est d'au moins 210 m²/g et, plus particulièrement, comprise entre 210 et 400 m²/g.

3. Silice selon la revendication 1 ou 2, caractérisée en ce que sa prise d'huile est comprise entre 220 et 280 ml/100 g.

4. Silice selon l'une des revendications précédentes, caractérisée en ce que son pH est compris entre 4 et 8.

5. Composition conditionnée caractérisée en ce qu'elle comprend un liquide absorbé sur un support comprenant une silice précipitée présentant les caractéristiques suivantes :
- surface BET d'au moins 170 m²/g ;
- prise d'huile (DOP) comprise entre 220 et 300 ml/100g ;
- densité de remplissage à l'état tassé d'au moins 0,29 ;
- diamètre médian des particules compris entre 80 et 150 µm ;
- un indice de dispersion granulométrique d'au plus 0,70.

6. Composition selon la revendication 5, caractérisée en ce que la silice présente une surface BET d'au moins 210 m²/g et, plus particulièrement, comprise entre 210 et 400 m²/g.

7. Composition selon la revendication 5 ou 6 caractérisée en ce que la silice présente une prise d'huile comprise entre 220 et 280 ml/100 g.

8. Composition selon l'une des revendications 5 à 7, caractérisée en ce que la silice présente un pH compris entre 4 et 8.

9. Composition selon l'une des revendications 5 à 8, caractérisée en ce que le liquide précité est une vitamine.

10. Composition selon la revendication 9, caractérisée en ce que le liquide précité est choisi dans le groupe comprenant les vitamines A, B, C, D, E et K.

11. Composition selon la revendication 9 ou 10, caractérisée en ce que le liquide précité est le chlorhydrate de choline.

12. Composition selon l'une des revendications 9 à 11, caractérisée en ce qu'elle présente une densité de remplissage à l'état tassé d'au moins 0,60, plus particulièrement d'au moins 0,65.

13. Composition selon l'une des revendications 9 à 12, caractérisée en ce qu'elle présente un angle de talus d'au plus 32°.

14. Procédé de préparation d'une silice du type selon l'une des revendications 1 à 4 caractérisé en ce qu'il comprend la réaction d'un silicate avec un agent acidificant ce par quoi l'on obtient une bouillie réactionnelle, la filtration de ladite bouillie ce par quoi l'on obtient un gâteau de filtration, le séchage dudit gâteau, ledit gâteau présentant avant séchage une perte au feu d'au plus 77 %, ledit séchage étant éventuellement précédé par un délitage du gâteau.

## Revendications (Revendications pour l'(les) Etat(s) contractant(s) suivant(s): ES)

1. Procédé de préparation d'une silice précipitée présentant les caractéristiques suivantes :
- surface BET d'au moins 170 m²/g ;
- prise d'huile (DOP) comprise entre 220 et 300 ml/100g ;
- densité de remplissage à l'état tassé d'au moins 0,29 ;
- diamètre médian des particules compris entre 80 et 150 µm ;
- un indice de dispersion granulométrique d'au plus 0,70,
ledit procédé comprenant la réaction d'un silicate avec un agent acidifiant ce par quoi l'on obtient une bouillie réactionnelle, la filtration de ladite boule ce par quoi l'on obtient un gâteau de filtration, le séchage dudit gâteau, ledit gâteau présentant avant séchage une perte au feu d'au plus 77 %, ledit séchage étant éventuellement précédé par un délitage du gâteau.

2. Procédé selon la revendication 1 caractérisée en ce que ladite surface BET est d'au moins 210 m²/g et, plus particulièrement, comprise entre 210 et 400 m²/g.

3. Procédé selon la revendication 1 ou 2, caractérisée en ce que ladite prise d'huile est comprise entre 220 et 280 ml/100 g.

4. Procédé selon l'une des revendications précédentes, caractérisée en ce que le pH de ladite silice est compris entre 4 et 8.

5. Composition conditionnée caractérisée en ce qu'elle comprend un liquide absorbé sur un support comprenant une silice précipitée présentant les caractéristiques suivantes :
- surface BET d'au moins 170 m²/g ;
- prise d'huile (DOP) comprise entre 220 et 300 ml/100g ;
- densité de remplissage à l'état tassé d'au moins 0,29 ;
- diamètre médian des particules compris entre 80 et 150 µm ;
- un indice de dispersion granulométrique d'au plus 0,70.

6. Composition selon la revendication 5, caractérisée en ce que la silice présente une surface BET d'au moins 210 m²/g et, plus particulièrement, comprise entre 210 et 400 m²/g.

7. Composition selon la revendication 5 ou 6 caractérisée en ce que la silice présente une prise d'huile comprise entre 220 et 280 ml/100 g.

8. Composition selon l'une des revendications 5 à 7, caractérisée en ce que la silice présente un pH compris entre 4 et 8.

9. Composition selon l'une des revendications 5 à 8, caractérisée en ce que le liquide précité est une vitamine.

10. Composition selon la revendication 9, caractérisée en ce que le liquide précité est choisi dans le groupe comprenant les vitamines A, B, C, D, E et K.

11. Composition selon la revendication 9 ou 10, caractérisée en ce que le liquide précité est le chlorhydrate de choline.

12. Composition selon l'une des revendications 9 à 11, caractérisée en ce qu'elle présente une densité de remplissage à l'état tassé d'au moins 0,60, plus particulièrement d'au moins 0,65.

13. Composition selon l'une des revendications 9 à 12, caractérisée en ce qu'elle présente un angle de talus d'au plus 32°.

## Claims (Claims for the following Contracting State(s): AT, BE, CH, DE, FR, GB, GR, IT, LI, LU, NL, SE)

1. A precipitated silica having the following characteristics:
- BET surface area of at least 170 m²/g;
- oil intake (DOP) of between 220 and 300 ml/100g;
- filling density in the compressed state of less than 0.29;
- mean particle diameter of between 80 and 150 µm;
- maximum granulometric dispersion index of 0.70.

2. A silica according to Claim 1, characterised in that its BET surface area is at least 210 m²/g, and, more particularly, between 210 and 400 m²/g.

3. A silica according to Claim 1 or Claim 2, characterised in that its oil intake is between 220 and 280 ml/100 g.

4. A silica according to one of the preceding Claims, characterised in that its pH value is between 4 and 8.

5. A conditioned composition, characterised in that it comprises a liquid absorbed on a support comprising a precipitated silica having the following characteristics:
- BET surface area of at least 170 m²/g;
- oil intake (DOP) of between 220 and 300 ml/100 g;
- filling density in the compressed state of at least 0.29;
- mean particle diameter of between 80 and 150 µm;
- maximum granulometric dispersion index of 0.70.

6. A composition according to Claim 5, characterised in that the silica has a BET surface area of at least 210 m²/g, and, more particularly, of between 210 and 400 m²/g.

7. A composition according to Claim 5 or Claim 6, characterised in that the silica has an oil intake of between 220 and 280 ml/100 g.

8. A composition according to one of Claims 5 to 7, characterised in that the silica has a pH value of between 4 and 8.

9. A composition according to one of Claims 5 to 8, characterised in that the aforesaid liquid is a vitamin.

10. A composition according to Claim 9, characterised in that the aforesaid liquid is selected from the group comprising vitamins A, B, C, D, E and K.

11. A composition according to Claim 9 or Claim 10, characterised in that the aforesaid liquid is choline hydrochloride.

12. A composition according to one of Claims 9 to 11, characterised in that it has a filling density in the compressed state of at least 0.60, more particularly of at least 0.65.

13. A composition according to one of Claims 9 to 12, characterised in that it has a slope angle greater than 32°.

14. A process for the preparation of a silica of the type according to any one of Claims 1 to 4, characterised in that it comprises reacting a silicate with an acidifying agent whereby a reactional mixture is obtained, said reactional mixture being filtered to obtain a filtration cake, said cake being dried, and said cake having, prior to drying, a combustion loss of above 77%, said drying operation possibly being preceded by slaking of the cake.

## Claims (Claims for the following Contracting State(s): ES)

1. A process for the preparation of a precipitated silica having the following characteristics:
- a BET surface area of at least 170 m²/g;
- an oil intake (DOP) of between 220 and 300 ml/100 g;
- a filling density in compressed state of at least 0.29;
- mean particle diameter of between 80 and 150 µm;
- granulometric dispersion index of above 0.70,
said process comprising the reaction of a silicate with an acidifying agent, whereby a reactional mixture is obtained, said mixture being filtered to obtain a filtration cake, said cake being dried, and said cake having, prior to drying, a maximum ignition loss of 77%, said drying operation possibly being preceded by slaking of the cake.

2. A process according to Claim 1, characterised in that said BET surface area is at least 210 m²/g, and, more particularly, between 210 and 400 m²/g.

3. A process according to Claim 1 or Claim 2, characterised in that said oil intake is between 220 and 280 ml/100 g.

4. A process according to one of the preceding claims, characterised in that the pH value of said silica is between 4 and 8.

5. A conditioned composition, characterised in that it comprises a liquid absorbed on a support containing a precipitated silica having the following characteristics:
- BET surface area of at least 170 m²/g;
- oil intake (DOP) of between 220 and 300 ml/100g;
- filling density in compressed state of at least 0.29;
- mean particle diameter of between 80 and 150 µm;
- maximum granulometric dispersion index of 0.70.

6. A composition according to Claim 5, characterised in that the silica has a BET surface area of at least 210 m²/g, and, more particularly, of between 210 and 400 m²/g.

7. A composition according to Claim 5 or Claim 6, characterised in that the silica has an oil intake of between 220 and 280 ml/100 g.

8. A composition according to one of Claims 5 to 7, characterised in that the silica has a pH value of between 4 and 8.

9. A composition according to one of Claims 5 to 8, characterised in that the aforesaid liquid is a vitamin.

10. A composition according to Claim 9, characterised in that the aforesaid liquid is selected from the group containing vitamins A, B, C, D, E and K.

11. A composition according to Claim 9 or Claim 10, characterised in that the aforesaid liquid is choline hydrochloride.

12. A composition according to one of Claims 9 to 11, characterised in that it has a filling density in compressed state of at least 0.60, more particularly of at least 0.65.

13. A composition according to one of Claims 9 to 12, characterised in that it has a maximum slope angle of 32°.

## Patentansprüche (Patentansprüche für folgende(n) Vertragsstaat(en): AT, BE, CH, DE, FR, GB, GR, IT, LI, LU, NL, SE)

1. Fällungskieselsäure, die die folgenden Eigenschaften aufweist:
- BET-Oberfläche von mindestens 170 m²/g;
- Ölaufnahme (DOP) eingeschlossen zwischen 220 und 300 ml/100 g;
- Fülldichte in gepreßtem Zustand von mindestens 0,29;
- mittlerer Teilchendurchmesser eingeschlossen zwischen 80 und 150 µm;
- ein granulometrischer Dispersionsindex von höchstens 0,70.

2. Kieselsäure nach Anspruch 1, dadurch gekennzeichnet, daß ihre BET-Oberfläche mindestens 210 m²/g beträgt und spezieller zwischen 210 und 400 m²/g eingeschlossen ist.

3. Kieselsäure nach Anspruch 1 oder 2, dadurch gekennzeichnet, daß ihre Ölaufnahme zwischen 220 und 280 ml/100 g eingeschlossen liegt.

4. Kieselsäure nach einem der vorangehenden Ansprüche, dadurch gekennzeichnet, daß ihr pH zwischen 4 und 8 eingeschlossen liegt.

5. Präparierte Zusammensetzung, dadurch gekennzeichnet, daß sie eine absorbierte Flüssigkeit auf einem Träger umfaßt, welcher eine die folgenden Eigenschaften aufweisende Fällungskieselsäure umfaßt:
- BET-Oberfläche von mindestens 170 m²/g;
- Ölaufnahme (DOP) eingeschlossen zwischen 220 und 300 ml/100 g;
- Fülldichte in gepreßtem Zustand von mindestens 0,29;
- mittlerer Teilchendurchmesser eingeschlossen zwischen 80 und 150 µm;
- ein granulometrischer Dispersionsindex von höchstens 0,70.

6. Zusammensetzung nach Anspruch 5, dadurch gekennzeichnet, daß die Kieselsäure eine BET-Oberfläche von mindestens 210 m²/g und spezieller zwischen 210 und 400 m²/g eingeschlossen aufweist.

7. Zusammensetzung nach Anspruch 5 oder 6, dadurch gekennzeichnet, daß die Kieselsäure eine zwischen 220 und 280 ml/100 g eingeschlossene Ölaufnahme aufweist.

8. Zusammensetzung nach einem der Ansprüche 5 bis 7, dadurch gekennzeichnet, daß die Kieselsäure einen zwischen 4 und 8 eingeschlossenen pH aufweist.

9. Zusammensetzung nach einem der Ansprüche 5 bis 8, dadurch gekennzeichnet, daß die vorgenannte Flüssigkeit ein Vitamin ist.

10. Zusammensetzung nach Anspruch 9, dadurch gekennzeichnet, daß die vorgenannte Flüssigkeit aus der die Vitamine A, B, C, D, E und K umfassenden Gruppe ausgewählt ist.

11. Zusammensetzung nach Anspruch 9 oder 10, dadurch gekennzeichnet, daß die vorgenannte Flüssigkeit Cholinhydrochlorid ist.

12. Zusammensetzung nach einem der Ansprüche 9 bis 11, dadurch gekennzeichnet, daß sie eine Fülldichte in gepreßtem Zustand von mindestens 0,60, spezieller von mindestens 0,65, aufweist.

13. Zusammensetzung nach einem der Ansprüche 9 bis 12, dadurch gekennzeichnet, daß sie einen Neigungswinkel von höchstens 32° aufweist.

14. Verfahren zur Herstellung einer Kieselsäure vom Typ gemäß einem der Ansprüche 1 bis 4, dadurch gekennzeichnet, daß es umfaßt die Umsetzung eines Silicats mit einem ansäuernden Mittel, wodurch man einen Reaktionsbrei erhält, die Filtration des Breis, wodurch man einen Filterkuchen erhält, das Trocknen des Kuchens, wobei der Kuchen vor dem Trocknen einen Glühverlust von höchstens 77% aufweist, wobei dem Trocknen gegebenenfalls ein Aufrühren des Kuchens vorausgeht.

## Patentansprüche (Patentansprüche für folgende(n) Vertragsstaat(en): ES)

1. Verfahren zur Herstellung einer Fällungskieselsäure, die die folgenden Eigenschaften aufweist:
- BET-Oberfläche von mindestens 170 m²/g;
- Ölaufnahme (DOP) eingeschlossen zwischen 220 und 300 ml/100 g;
- Fülldichte in gepreßtem Zustand von mindestens 0,29;
- mittlerer Teilchendurchmesser eingeschlossen zwischen 80 und 150 µm;
- ein granulometrischer Dispersionsindex von höchstens 0,70,
wobei das Verfahren umfaßt die Umsetzung eines Silicats mit einem ansäuernden Mittel, wodurch man einen Reaktionsbrei erhält, die Filtration des Breis, wodurch man einen Filterkuchen erhält, das Trocknen des Kuchens, wobei der Kuchen vor dem Trocknen einen Glühverlust von höchstens 77% aufweist, wobei dem Trocknen gegebenenfalls ein Aufrühren des Kuchens vorausgeht.

2. Verfahren nach Anspruch 1, dadurch gekennzeichnet, daß die BET-Oberfläche mindestens 210 m²/g beträgt und spezieller zwischen 210 und 400 m²/g eingeschlossen ist.

3. Verfahren nach Anspruch 1 oder 2, dadurch gekennzeichnet, daß die Ölaufnahme zwischen 220 und 280 ml/100 g eingeschlossen liegt.

4. Verfahren nach einem der vorangehenden Ansprüche, dadurch gekennzeichnet, daß der pH der Kieselsäure zwischen 4 und 8 eingeschlossen liegt.

5. Präparierte Zusammensetzung, dadurch gekennzeichnet, daß sie eine absorbierte Flüssigkeit auf einem Träger umfaßt, welcher eine die folgenden Eigenschaften aufweisende Fällungskieselsäure umfaßt:
- BET-Oberfläche von mindestens 170 m²/g;
- Ölaufnahme (DOP) eingeschlossen zwischen 220 und 300 ml/100 g;
- Fülldichte in gepreßtem Zustand von mindestens 0,29;
- mittlerer Teilchendurchmesser eingeschlossen zwischen 80 und 150 µm;
- ein granulometrischer Dispersionsindex von höchstens 0,70.

6. Zusammensetzung nach Anspruch 5, dadurch gekennzeichnet, daß die Kieselsäure eine BET-Oberfläche von mindestens 210 m²/g und spezieller zwischen 210 und 400 m²/g eingeschlossen aufweist.

7. Zusammensetzung nach Anspruch 5 oder 6, dadurch gekennzeichnet, daß die Kieselsäure eine zwischen 220 und 280 ml/100 g eingeschlossene Ölaufnahme aufweist.

8. Zusammensetzung nach einem der Ansprüche 5 bis 7, dadurch gekennzeichnet, daß die Kieselsäure einen zwischen 4 und 8 eingeschlossenen pH aufweist.

9. Zusammensetzung nach einem der Ansprüche 5 bis 8, dadurch gekennzeichnet, daß die vorgenannte Flüssigkeit ein Vitamin ist.

10. Zusammensetzung nach Anspruch 9, dadurch gekennzeichnet, daß die vorgenannte Flüssigkeit aus der die Vitamine A, B, C, D, E und K umfassenden Gruppe ausgewählt ist.

11. Zusammensetzung nach Anspruch 9 oder 10, dadurch gekennzeichnet, daß die vorgenannte Flüssigkeit Cholinhydrochlorid ist.

12. Zusammensetzung nach einem der Ansprüche 9 bis 11, dadurch gekennzeichnet, daß sie eine Fülldichte in gepreßtem Zustand von mindestens 0,60, spezieller von mindestens 0,65, aufweist.

13. Zusammensetzung nach einem der Ansprüche 9 bis 12, dadurch gekennzeichnet, daß sie einen Neigungswinkel von höchstens 32° aufweist.
